(19) Europäisches Patentamt
European Patent Office
Office européen des brevets

(11) Veröffentlichungsnummer: **0 391 108 B1**

(12) **EUROPÄISCHE PATENTSCHRIFT**

(45) Veröffentlichungstag der Patentschrift: **18.01.95**

(51) Int. Cl.$^{6}$: **C08F 283/06**, A61L 15/00

(21) Anmeldenummer: **90104995.7**

(22) Anmeldetag: **16.03.90**

# (54) Hydrophile, quellfähige Pfropfcopolymerisate, deren Herstellung und Verwendung.

(30) Priorität: **01.04.89 DE 3910563**

(43) Veröffentlichungstag der Anmeldung:
**10.10.90 Patentblatt 90/41**

(45) Bekanntmachung des Hinweises auf die Patenterteilung:
**18.01.95 Patentblatt 95/03**

(84) Benannte Vertragsstaaten:
**BE CH DE DK ES FR GB IT LI NL SE**

(56) Entgegenhaltungen:
**EP-A- 0 316 792**
**DE-A- 3 401 813**

(73) Patentinhaber: **CASSELLA Aktiengesellschaft**
**Hanauer Landstrasse 526**
**D-60386 Frankturt (DE)**

(72) Erfinder: **Engelhardt, Friedrich, Dr.**
**Hünfelder Strasse 20**
**D-6000 Frankturt am Main 61 (DE)**
Erfinder: **Riegel, Ulrich**
**Steinäckerstrasse 6**
**D-6000 Frankturt am Main 61 (DE)**
Erfinder: **Kühlwein, Jürgen, Dr.**
**Mainstrasse 21**
**D-6050 Offenbach/Main (DE)**

(74) Vertreter: **Muley, Ralf, Dr.**
**Cassella AG,**
**Patentabteilung,**
**Hanauer Landstrasse 526**
**D-60386 Frankturt (DE)**

**EP 0 391 108 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft hydrophile, quellfähige Pfropfpolymerisate, die in statistischer Verteilung zu 0,5 bis 20 Gew.% aus Resten der allgemeinen Formel I

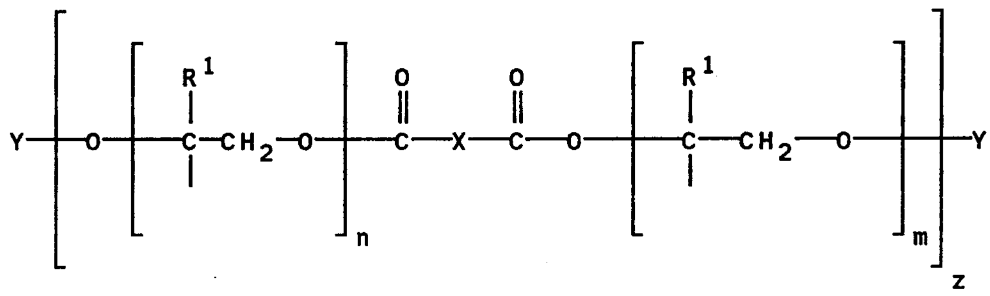

(I)

zu 79 bis 99 Gew.% aus eine saure Gruppe enthaltenden Resten der allgemeinen Formel II

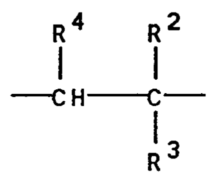

(II)

und zu 0,1 bis 2 Gew.% aus Resten eines Vernetzers, die aus Monomeren mit mindestens zwei olefinisch ungesättigten Doppelbindungen hervorgegangen sind, bestehen, wobei X ($C_1$-$C_6$)-Alkylen, ($C_2$-$C_6$)-Alkenylen, Phenylen oder durch Sulfonyl substituiertes Phenylen, wobei cis-Ethenylen ausgeschlossen ist,
Y Wasserstoff oder

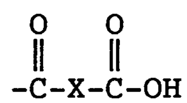

,

z 1 bis 100,
m und n unabhängig voneinander 2 bis 300,
$R^1$ Wasserstoff oder Methyl,
$R^2$ Wasserstoff, Methyl oder Ethyl,
$R^3$ die Carboxylgruppe, die Sulfonylgruppe, die Phosphonylgruppe, die gegebenenfalls mit Alkanol mit 1 bis 4 Kohlenstoffatomen verestert sein kann, oder eine Gruppe der Formel

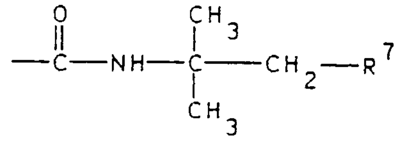

worin $R^7$ für die Sulfonylgruppe oder die Phosphonylgruppe steht, $R^4$ Wasserstoff, Methyl, Ethyl oder die Carboxylgruppe bedeuten, sowie ihre Herstellung und Verwendung als Absorptionsmittel für Wasser und wäßrige Lösungen, zum Beispiel in Hygieneartikeln, zur Bodenverbesserung oder als Filtrationshilfsmittel.

Quellbare Polymere, die wäßrige Lösungen absorbieren, werden für die Herstellung von Tampons, Windeln, Damenbinden und anderen Hygieneartikeln sowie als wasserzurückhaltende Mittel im landwirtschaftlichen Gartenbau verwendet.

Zu bekannten Absorptionsharzen dieses Typs gehören vernetzte Carboxymethylcellulose, teilweise vernetztes Polyalkylenoxid, Hydrolysate von Stärke-Acrylnitril-Pfropfcopolymeren oder teilweise vernetzte

Polyacrylsäuresalze.

Diese bekannten Polymerisate zeigen durchweg Nachteile, insbesondere bei der Absorption wäßriger Elektrolytlösung sowie Blut und Urin.

Bei hohem Absorptionsvermögen werden nach dem derzeitigen Stand der Technik zu geringe Gelfestigkeiten der gequollenen Polymerpartikel erreicht. Es bilden sich klebrige Massen, welche die Saugfähigkeit der damit hergestellten Produkte verschlechtern.

Es ist bekannt, daß durch Erhöhung der Vernetzungsdichte die Gelfestigkeit sowie die Geschwindigkeit der Flüssigkeitsaufnahme erhöht werden kann, dadurch jedoch gleichzeitig die Absorptionskapazität herabgesetzt wird. Diese Vorgehensweise ist insofern unerwünscht als die Absorptionskapazität die wichtigste Eigenschaft des Polymeren ist.

Aufgabe der vorliegenden Erfindung ist es, modifizierte Polymere, die wäßrige Lösungen absorbieren, bereitzustellen, welche eine hohe Absorptionsrate aufweisen und dabei im gequollenen Zustand nicht klebende Hydrogelpartikel hoher Gelfestigkeit bilden.

Überraschenderweise wurde nun gefunden, daß das gewünschte Eigenschaftsprofil durch die erfindungsgemäßen Pfropfpolymerisate erreicht wird, da deren makromolekulares Netzwerk physikalisch eine Erhöhung der Gelfestigkeit oder Gelstärke des gequollenen Polymeren sowie eine verbesserte Elektrolyttoleranz bewirkt.

Bevorzugte erfindungsgemäße Produkte bestehen zu 0,5 bis 15 Gew.% aus Resten der allgemeinen Formel I, 84 bis 99 Gew.% aus Resten der allgemeinen Formel II und 0,1 bis 1,8 Gew.% aus vernetzenden Strukturen, die aus Monomeren mit mindestens zwei olefinisch ungesättigten Doppelbindungen hervorgegangen sind.

Besonders bevorzugte erfindungsgemäße Produkte bestehen zu 1 bis 10,5 Gew.% aus Resten der allgemeinen Formel I, 88 bis 98,5 Gew.% aus Resten der allgemeinen Formel II und 0,3 bis 1,5 Gew.% aus vernetzenden Strukturen, die aus Monomeren mit mindestens zwei olefinisch ungesättigten Doppelbindungen hervorgegangen sind.

In den erfindungsgemäßen Pfropfcopolymerisation können die Reste der allgemeinen Formel I alle exakt die gleiche Struktur haben, sie können sich jedoch auch hinsichtlich des Restes $R^1$ und/oder der Zahlen m und n voneinander unterscheiden. So können sich bezüglich $R^1$ Wasserstoff und Methyl in statistischer Weise abwechseln, es können aber auch größere Polymerabschnitte aufeinander folgen, in denen $R^1$ jeweils nur Wasserstoff oder nur Methyl bedeutet.

Die Diolkomponenten in den Resten der allgemeinen Formel I leiten sich bevorzugt ab von Polyethylenglykolen mit einem Molgewicht von 100 bis 8.000, Polypropylenglykolen mit einem Molgewicht von 140 bis 8.000, statistisch aus Ethylenglykol- und Propylenglykoleinheiten aufgebauten Produkten mit einem Molgewicht von 130 bis 10.000 und von Blockcopolymeren, aufgebaut aus Ethylenglykol- und Propylenglykoleinheiten mit einem Molgewicht von 130 bis 10.000.

X bedeutet bevorzugt ($C_1$-$C_6$)-Alkylen, 1,4-Phenylen, 1,3-Phenylen oder 5-Sulfo-1,3-phenylen.

z bedeutet bevorzugt 2 bis 50.

In den Resten der allgemeinen Formel II bedeutet $R^2$ bevorzugt Wasserstoff oder Methyl. $R^3$ steht bevorzugt für die Carboxylgruppe, die Sulfonylgruppe oder die Phosphonylgruppe. Besonders bevorzugt ist die Carboxylgruppe. $R^4$ bedeutet bevorzugt Wasserstoff.

Die genannten vernetzenden Strukturen können sich von allen geeigneten Monomeren mit mindestens zwei olefinisch ungesättigten Doppelbindungen ableiten.

Geeignete Monomere sind beispielsweise Verbindungen, die mindestens zwei Alkenylgruppen, zum Beispiel Vinyl oder Allyl, oder mindestens zwei Alkenoylgruppen, zum Beispiel Acrylat oder Methacrylat, enthalten.

Bevorzugt leiten sich die vernetzenden Strukturen von Monomeren ab, die 2, 3 oder 4 ethylenisch ungesättigte Doppelbindungen enthalten.

Besonders bevorzugt leiten sich die vernetzenden Strukturen von Trimethylolpropantriacrylat, Tetraallyloxyethan oder Methylenbisacrylamid ab.

Ganz besonders bevorzugte erfindungsgemäße Pfropfpolymerisate sind solche, in denen mehrere der oben genannten bevorzugten oder besonders bevorzugten Merkmale enthalten sind.

Die erfindungsgemäßen Pfropfpolymerisate können durch bekannte Polymerisationsverfahren hergestellt werden. Bevorzugt ist die Polymerisation in wäßriger Lösung nach dem Verfahren der sogenannten Gelpolymerisation. Dabei werden 15-50%ige wäßrige Lösungen der Comonomeren mit bekannten geeigneten Katalysatorsystemen ohne mechanische Durchmischung unter Ausnutzung des Trommsdorff-Norrish-Effektes (Bios Final Rep. 363.22; Makromol. Chem. 1, 169 (1947)) polymerisiert.

Die Polymerisationsreaktion kann im Temperaturbereich zwischen 0°C und 130°C, vorzugsweise zwischen 10°C und 100°C, sowohl bei Normaldruck als auch unter erhöhtem Druck durchgeführt werden. Wie

üblich kann die Polymerisation, auch in einer Schutzgasatmosphäre, vorzugsweise unter Stickstoff, ausgeführt werden.

Zur Auslösung der Polymerisation können energiereiche - elektromagnetische Strahlen oder die üblichen chemischen Polymerisationsinitiatoren herangezogen werden, z.B. organische Peroxide, wie Benzoylperoxid, tert. Butylhydroperoxid, Methyl-ethyl-keton-peroxid, Cumol-hydroperoxid, Azoverbindungen wie Azo-di-iso-butyro-nitril sowie anorganische Peroxiverbindungen wie $(NH_4)_2S_2O_8$ oder $K_2S_2O_8$ oder $H_2O_2$ gegebenenfalls in Kombination mit Reduktionsmitteln wie Natriumhydrogensulfit, und Eisen-II-Sulfat oder Redoxsysteme, welche als reduzierende Komponente eine aliphatische und aromatische Sulfinsäure, wie Benzolsulfinsäure und Toluolsulfinsäure oder Derivate dieser Säuren, enthalten, wie z.B. Mannichaddukte aus Sulfinsäure, Aldehyden und Aminoverbindungen, wie sie in der deutschen Patentschrift 1301516 beschrieben sind. Pro 100 g Gesamtmonomeren werden in der Regel 0,03 bis 2 g des Polymerisationsinitiators eingesetzt.

Durch mehrstündiges Nachheizen der Polymerisatgele im Temperaturbereich von 50-130°C, vorzugsweise 70-100°C, können die Qualitätseigenschaften der Polymerisate noch verbessert werden.

Die auf diesem Wege hergestellten, in Form wäßriger Gallerten vorliegenden erfindungsgemäßen Copolymerisate können nach mechanischer Zerkleinerung mit geeigneten Apparaten durch bekannte Trocknungsverfahren in fester Form erhalten werden und zum Einsatz gelangen.

Zweckmäßigerweise werden somit erfindungsgemäße Pfropfpolymerisate erhalten, wenn 0,5 bis 20 Gew.%, vorzugsweise 0,5 bis 15, insbesondere 1 bis 10,5 Gew.%, einer Polyalkylenoxidverbindung der allgemeinen Formel Ia

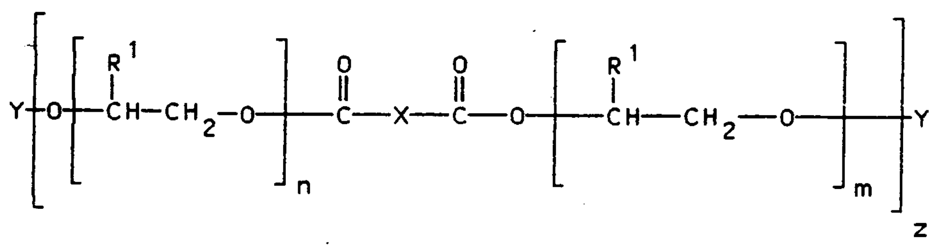 (Ia)

79 bis 99 Gew.%, vorzugsweise 84 bis 99, insbesondere 88 bis 98,5 Gew.%, einer ungesättigten Säure der allgemeinen Formel IIa

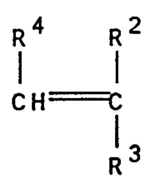 (IIa)

oder ein Alkali-, Ammonium- oder Aminsalz davon und 0,1 bis 2 Gew.%, vorzugsweise 0,1 bis 1,8, insbesondere 0,3 bis 1,5 Gew.%, eines Monomeren mit mindestens zwei olefinisch ungesättigten Doppelbindungen, wobei die Reste $R^1$ bis $R^4$, X, Y und die Zahlen m, n und z die oben genannten Bedeutungen haben, unter den Bedingungen der Gelpolymerisation umgesetzt werden.

Die Polyalkylenoxidverbindungen der allgemeinen Formel Ia können durch eine einfache Veresterungsreaktion zwischen Säuren der allgemeinen Formel III

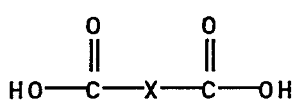 (III)

worin X wie oben definiert ist, oder geeigneten Derivaten davon und Polyalkylenoxiden erhalten werden. Geeignete Polyalkylenoxide sind zum Beispiel Polyethylenglykol, Polypropylenglykol, Blockcopolymere aus Polyethylenoxid- und Polypropylenoxidblöcken sowie statistische Ethylen/Propylenoxid-Copolymere.

Die Monomeren der Formel IIa sind bekannte Verbindungen, wie zum Beispiel Acrylsäure, Methacrylsäure, Vinylsulfonsäure, Fumarsäure, Crotonsäure, 2-Acrylamido-2-methylpropansulfonsäure, 2-Acrylamido-2-methyl-propanphosphonsäure und Vinylphosphonsäure sowie deren Halbester.

Die als Vernetzer eingesetzten polyolefinischen Monomere sind gängige Produkte. Beispiele sind Bisacrylamidoessigsäure, Trimethylolpropantriacrylat und Tetraallyloxyethan.

Die erfindungsgemäßen Pfropfpolymerisate eignen sich hervorragend als Absorptionsmittel für Wasser und wäßrige Lösungen, so daß sie vorteilhaft als Wasser zurückhaltendes Mittel im landwirtschaftlichen Gartenbau, als Filtrationshilfsmittel und besonders als saugfähige Komponente in Hygieneartikeln wie Windeln, Tampons oder Damenbinden eingesetzt werden können.

Die folgenden Beispiele veranschaulichen die Herstellung erfindungsgemäßer Pfropfpolymerisate.

Beispiel 1:

In einen durch geschäumtes Kunststoffmaterial gut isolierten Polyethyleneimer mit einem Fassungsvermögen von 10 l werden 5169 g entsalztes Wasser vorgelegt, 1000 g Natriumbicarbonat darin dispergiert und langsam 1888 g Acrylsäure so zudosiert, daß ein Überschäumen der Reaktionslösung vermieden wird, wobei sich diese auf eine Temperatur von ca. 12 - 10°C abkühlt. Es werden nun 100 g des als Pfropfgrundlage dienenden Umsetzungsproduktes gemäß Beispiel a (siehe unten), 12 g Trimethylolpropantriacrylat und 10 g eines Natrium-Diisooctylsulfosuccinates (Rewopol V 2133 der Firma REWO, Steinau) zugegeben. Bei einer Temperatur von 10 - 12°C werden die Initiatoren, ein Redoxsystem, bestehend aus 2,2 g 2,2'-Azobisamidinopropan-Dihydrochlorid, gelöst in 20 g Wasser, 4,4 g Kaliumperoxidsulfat, gelöst in 170 g Wasser, und 6 g Natriumpyrosulfit, gelöst in 120 g Wasser, nacheinander zugegeben und gut verrührt. Die Reaktionslösung wird daraufhin ohne Rühren stehen gelassen, wobei durch einsetzende Polymerisation, in dessen Verlauf die Temperatur bis auf ca. 85°C ansteigt, ein festes Gel entsteht. Dieses wird anschließend mechanisch zerkleinert, bei Temperaturen über 80°C getrocknet und gemahlen.

Das vorstehend beschriebene Produkt wurde in herkömmlicher Weise in eine Babywindel eingearbeitet und zeichnete sich hier durch eine besonders gute Flüssigkeitsretention aus.

Herstellung der als Pfropfgrundlage dienenden hydrophilen Polyester:

Beispiel a

Verfahren A:

In einem 4-Liter-Kolben werden 2025 g (1,35 mol) Polyethylenglykol 1500 aufgeschmolzen und dann unter Rühren mit 513,7 g (6,75 mol) Propandiol-1,2, 786,5 g (4.05 mol) Dimethylterephthalat und 1,15 g (0,004 mol) Titan-IV-isopropylat versetzt. Das Reaktionsgemisch wird nun unter Rühren und Überleiten eines schwachen Stickstoffstromes auf 180°C erwärmt. Das bei der jetzt einsetzenden Reaktion entstehende Methanol wird über eine Füllkörperkolonne mit Destillationsaufsatz abdestilliert. Die Reaktionstemperatur wird solange gesteigert, bis 328 ml Methanol angefallen sind.

Nun wird die Füllkörperkolonne gegen einen absteigenden Kühler mit graduierter Vorlage ausgetauscht. Bei 230°C wird dann der Druck innerhalb von 2 - 3 Stunden auf 60 mbar reduziert und soviel überschüssiges Propandiol-1,2 abdestilliert, bis die Reaktionsmasse eine OH-Zahl von 53 und eine Carboxylzahl von 1 oder kleiner aufweist.

Beispiel b

Verfahren B:

In einem 4-Liter-Kolben werden 2228,6 g (21 mol) Diethylenglykol und 600 g (1 mol) Polyethylenglykol 600 bei 100°C vorgelegt und dann unter Rühren mit 830,6 g (5 mol) Isophthalsäure, 664,6 g (4 mol) Terephthalsäure, 268,2 g 5-Sulfoisophthalsäure Na-Salz (SIA) (1 mol) und 2,68 g (1 Gew.-% bzgl. SIA) wasserfreier Soda versetzt. Das Reaktionsgemisch wird nun unter Rühren und Überleiten eines schwachen Stickstoffstromes auf 190 °C erwärmt. Das bei der jetzt einsetzenden Reaktion entstehende Wasser wird über eine Füllkörperkolonne mit Destillationsaufsatz abgetrennt. Die Reaktionstemperatur wird solange gesteigert, bis 360 g Wasser abdestilliert sind. Nun wird die Füllkörperkolonne gegen einen absteigenden Kühler mit graduierter Vorlage ausgetauscht. Nach Anlegen von 1 mbar wird die Temperatur innerhalb von 3 Stunden auf 280°C gesteigert und dabei solange gehalten, bis die Reaktionsmasse eine OH-Zahl von 15

und eine Carboxylzahl von 2 bis 3 aufweist.

Beispiel c

Verfahren C:

In einem 0,5-Liter-Kolben werden 300 g (0,1 mol) Polyethylenglykol 3000 aufgeschmolzen und dann unter Rühren mit 44,04 g Polybutylenterephthalat-1,4 und 57 mg (0,0002 mol) Titan-IV-isopropylat versetzt. Das Reaktionsgemisch wird nun unter Rühren und Überleiten eines schwachen Stickstoffstromes auf 260°C erwärmt. Es wird nun solange bei diesen Bedingungen gehalten, bis die dabei ablaufende Depolymerisation zu einer vollständigen Auflösung des Polybutylenterephthalats geführt hat, und die Reaktionsmasse eine OH-Zahl von 32 und eine Carboxylzahl von 2 und kleiner aufweist.

Beispiel d

Verfahren D:

Dieses Verfahren ist als Azeotrop-Veresterung insbesondere zur Herstellung von Polykondensationsverbindungen aus oxalkylierten Fettaminen, Diolen und aliphatischen Dicarbonsäuren geeignet und ist aus den beiden Druckschriften DE 35 26 600-A1 und DE 35 26 601-A1 bekannt.

In der folgenden Tabelle 1 sind weitere Pfropfgrundlagen aufgeführt.

Tabelle 1

| Polykondensat Beisp. | Polydiol Molgewichtsanteile | | Diol | Molverhältnis Diol: Polydiol | Dicarbonsäure | OH-Zahl | Carboxyl-Zahl | Verfahren |
|---|---|---|---|---|---|---|---|---|
| | POE | POP | | | | | | |
| e | 1500 | - | PPD-1.2 | 3 : 1 | TPA | 53 | 0,6 | A |
| f | 850 | 1750 | DEG | 3 : 1 | TPA | 30 | 1,5 | A |
| g | 1750 | 1750 | PPD-1.2 | 3 : 1 | TPA | 27 | 0,8 | A |
| h | 3800 | 950 | MEG | 2,5:1 | TPA | 19 | 2,5 | A |
| i | 3000 | - | MEG | 3 : 1 | TPA | 28 | 4 | A |
| j | 600 | - | DEG | 9 : 1 | TPA/IPA/SIA 4 : 5 : 1 | 15 | 2,5 | B |
| k | 300 | 300 | DEG | 9 : 1 | TPA/IPA/SIA 4 : 5 : 1 | 12 | 2 | B |
| l | 3000 | - | BD-1.4 | 2 : 1 | TPA | 32 | 2 | C |
| m | 3800 | 950 | MEG | 2,5:1 | TPA | 20 | 1,5 | C |

POE = Polyoxyethylen
POP = Polyoxypropylen
PPD-1.2 = Propandiol-1.2
DEG = Diethylenglykol
MEG = Monoethylenglykol
BD-1.4 = Butandiol-1.4
TPA = Terephthalsäure
IPA = Isophthalsäure
SIA = 5-Sulfoisophthalsäure

Beispiel 2:

In einem 10 Liter-Kunststoffeimer werden 4419 g Eis und 1888 g Acrylsäure vorgelegt und langsam 1573 g NaOH 50%ig zudosiert, anschließend 100 g des als Pfropfgrundlage dienenden Umsetzungsproduktes gemäß Beispiel 1b, 12 g Bisacrylamidoessigsäure, dispergiert in 100 g Wasser und durch Zugabe von NaOH gelöst und auf pH 6 eingestellt, und 10 g Rewopol V 2133 zugegeben. Die Reaktionslösung wird auf 20°C eingestellt und anschließend mit den Initiatoren, ein Redoxsystem bestehend aus 6 g Kaliumperoxidisulfat, gelöst in 170 g Wasser, und 0,15 g Ascorbinsäure, gelöst in 120 g Wasser, versetzt und ohne Rühren stehen gelassen. Das durch Polymerisation entstehende Gel wird anschließend mechanisch zerkleinert, bei Temperaturen über 80°C getrocknet und gemahlen.

Beispiel 3:

In einem 10 Liter-Polyethyleneimer werden 5250 g entsalztes Wasser, 1888 g Acrylsäure und 100 g des als Pfropfgrundlage dienenden Umsetzungsproduktes gemäß Beispiel 1c vorgelegt. Es werden 12 g Tetraallyloxyethan eingerührt. Nach Einstellen der Reaktionslösung auf 18 - 20°C werden die Initiatoren, 6 g Kaliumperoxidisulfat in 170 g Wasser und 0,2 g Ascorbinsäure in 20 g Wasser nacheinander zugegeben und das Reaktionsgefäß wird gut isoliert ohne Rühren stehen gelassen. Nach einsetzender Reaktion steigt die Temperatur bis auf ca. 90°C an, und es entsteht ein festes Gel. Dieses wird mechanisch durch einen Extruder zerkleinert, dem kontinuierlich 1555 g NaOH 50%ig zudosiert werden, wobei teilweise Verdampfung des Wassers erfolgt. Das flockige Polymer wird anschließend bei Temperaturen über 80°C endgetrocknet und gemahlen.

Weitere Beispiele zur Herstellung erfindungsgemäßer Pfropfpolymerisate gemäß der hier beschriebenen Beispiele 1 und 2 sind in folgender Tabelle zusammengefaßt. Die Mengenangaben bedeuten Gewichts% bezogen auf Gesamtmonomeranteil.

Folgende Abkürzungen werden benutzt:

| | |
|---|---|
| AS: | Acrylsäure |
| MAS: | Methacrylsäure |
| CTS: | Crotonsäure |
| VPS: | Vinylphosphonsäure |
| VPE: | Vinylphosphonsäurehalbester |
| AMP: | 2-Acrylamido-2-methyl-propansulfonsäure |
| AMPP: | 2-Acrylamido-2-methyl-propanphosphonsäure |
| BAAE: | Bisacrylamidoessigsäure |
| TMPTA: | Trimethylolpropantriacrylat |
| TAE: | Tetraallyloxyethan |

| Beispiel | hergestellt analog Beispiel | AS (%) | MAS (%) | AMP (%) | AMPP (%) | VPS (%) | VPE (%) | CTS (%) | Pfropfgrundlage gemäß Beispiel | Pfropfgrundlage (%) | BAAE (%) | TMPTA (%) | TAE (%) | Neutralisationsgrad (%) |
|---|---|---|---|---|---|---|---|---|---|---|---|---|---|---|
| 4 | 1 | 94,4 | | | | | | | 1d | 5 | | 0,6 | | 45 |
| 5 | 1 | 89,4 | | | | | | | 1e | 10 | | 0,6 | | 45 |
| 6 | 1 | 94,4 | | | | | | | 1f | 5 | | 0,6 | | 45 |
| 7 | 1 | 94,4 | | | | | | | 1g | 5 | | 0,6 | | 45 |
| 8 | 1 | 94,4 | | | | | | | 1h | 5 | | 0,6 | | 45 |
| 9 | 1 | 88,5 | | | | | | | 1i | 10 | | 1,5 | | 70 |
| 10 | 1 | 94,4 | | | | | | | 1j | 5 | | 0,6 | | 45 |
| 11 | 2 | 89,4 | | | | | | | 1k | 10 | 0,6 | | | 75 |
| 12 | 2 | 89,4 | | | | | | | 1l | 10 | | | 0,6 | 78 |
| 13 | 1 | 98,4 | | | | | | | 1m | 1 | | 0,6 | | 45 |
| 14 | 2 | 85,0 | | 5,0 | 4,5 | | | | 1a | 5 | 0,5 | | | 70 |
| 15 | 1 | 72,0 | | 20,0 | | 4,2 | | | 1a | 3 | 0,8 | | | 80 |
| 16 | 1 | 81,0 | 10,0 | | | | 4,0 | | 1a | 4 | | 1,0 | | 36 |
| 17 | 2 | 90,0 | | | 4,6 | | | | 1a | 5 | | | 0,4 | 25 |
| 18 | 2 | 79,0 | | 19,0 | | | | | 1a | 1 | | | 1,0 | 40 |
| 19 | 1 | 72,0 | | 19,3 | | | | 5,0 | 1a | 3 | | | 0,7 | 48 |
| 20 | 1 | 90,0 | | | | 1,0 | | | 1a | 8 | | | 1,0 | 32 |

**Patentansprüche**

**Patentansprüche für folgende Vertragsstaaten : BE, CH, DE, DK, FR, GB, IT, LI, NL, SE**

1. Hydrophile, quellfähige Pfropfpolymerisate, die in statistischer Verteilung zu 0,5 bis 20 Gew.% aus Resten der allgemeinen Formel I

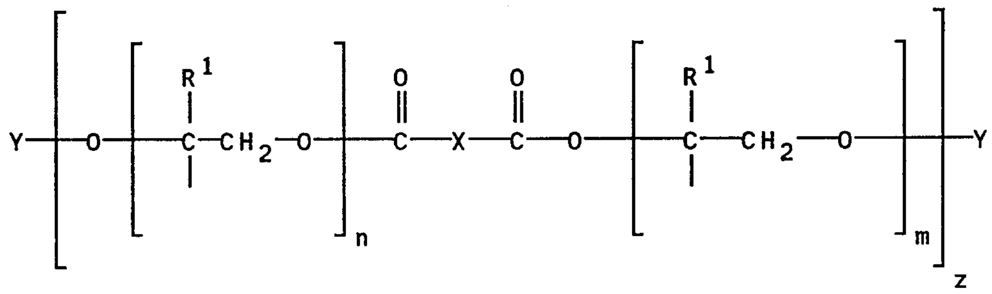

(I)

zu 79 bis 99 Gew.% aus eine saure Gruppe enthaltenden Resten der allgemeinen Formel II

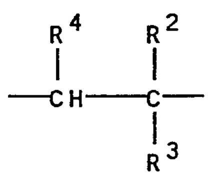

(II)

und zu 0,1 bis 2 Gew.% aus Resten eines Vernetzers, die aus Monomeren mit mindestens zwei olefinisch ungesättigten Doppelbindungen hervorgegangen sind, bestehen, wobei X ($C_1$-$C_6$)-Alkylen, ($C_2$-$C_6$)-Alkenylen, Phenylen oder durch Sulfonyl substituiertes Phenylen, wobei cis-Ethenylen ausgeschlossen ist,
Y Wasserstoff oder

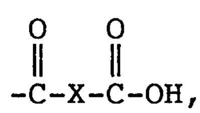

z 1 bis 100,
m und n unabhängig voneinander 2 bis 300,
$R^1$ Wasserstoff oder Methyl,
$R^2$ Wasserstoff, Methyl oder Ethyl,
$R^3$ die Carboxylgruppe, die Sulfonylgruppe, die Phosphonylgruppe, die gegebenenfalls mit Alkanol mit 1 bis 4 Kohlenstoffatomen verestert sein kann, oder eine Gruppe der Formel

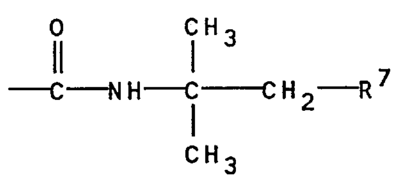

worin $R^7$ für die Sulfonylgruppe oder die Phosphonylgruppe steht, $R^4$ Wasserstoff, Methyl, Ethyl oder die Carboxylgruppe bedeuten.

**2.** Pfropfpolymerisate gemäß Anspruch 1, dadurch gekennzeichnet, daß sie zu 1 bis 10,5 Gew.% aus Resten der allgemeinen Formel I, zu 88 bis 98,5 Gew.% aus Resten der allgemeinen Formel II und zu 0,3 bis 1,5 Gew.% aus vernetzenden Strukturen bestehen.

**3.** Pfropfpolymerisate gemäß Ansprüchen 1 und/oder 2, dadurch gekennzeichnet, daß sich die Reste der allgemeinen Formel I hinsichtlich des Restes $R^1$ und/oder der Zahlen m und n voneinander unterscheiden.

**4.** Pfropfpolymerisate nach einem oder mehreren der Ansprüche 1 bis 3, dadurch gekennzeichnet, daß in den Resten der allgemeinen Formel II $R^2$ Wasserstoff oder Methyl, $R^3$ die Carboxylgruppe, die Sulfonylgruppe oder die Phosphonylgruppe und $R^4$ Wasserstoff bedeuten.

**5.** Pfropfpolymerisate nach einem oder mehreren der Ansprüche 1 bis 4, dadurch gekennzeichnet, daß in den Resten der allgemeinen Formel II $R^3$ die Carboxylgruppe bedeutet.

**6.** Pfropfpolymerisate nach einem oder mehreren der Ansprüche 1 bis 5, dadurch gekennzeichnet, daß sich die vernetzenden Strukturen von Monomeren mit mindestens zwei Alkenylgruppen oder mindestens zwei Alkenoylgruppen, insbesondere von Trimethylolpropantriacrylat, Tetraallyloxyethan oder Methylenbisacrylamid ableiten.

**7.** Pfropfpolymerisate nach einem oder mehreren der Ansprüche 1 bis 6, dadurch gekennzeichnet, daß X $(C_1\text{-}C_6)$-Alkylen, 1,4-Phenylen, 1,3-Phenylen oder 5-Sulfo-1,3-phenylen bedeutet.

**8.** Verfahren zur Herstellung der in den Ansprüchen 1 bis 7 beanspruchten Pfropfpolymerisate, dadurch gekennzeichnet, daß 0,5 bis 20 Gew.%, vorzugsweise 0,5 bis 15, insbesondere 1 bis 10,5 Gew.%, einer Polyalkylenoxidverbindung der allgemeinen Formel Ia

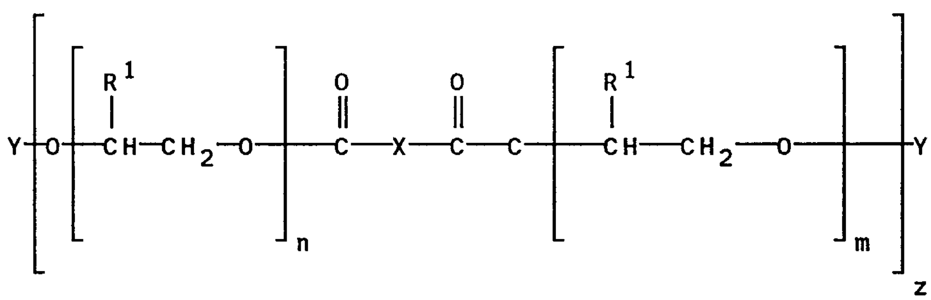

(Ia)

79 bis 99 Gew.%, vorzugsweise 84 bis 99, insbesondere 88 bis 98,5 Gew.%, einer ungesättigten Säure der allgemeinen Formel IIa

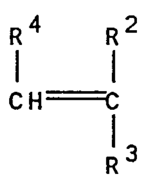

(IIa)

oder ein Alkali-, Ammonium- oder Aminsalz davon und 0,1 bis 2 Gew.%, vorzugsweise 0,1 bis 1,8, insbesondere 0,3 bis 1,5 Gew.%, eines Monomeren mit mindestens zwei olefinisch ungesättigten Doppelbindungen, wobei die Reste $R^1$ bis $R^4$, X, Y und die Zahlen m, n und z die oben genannten Bedeutungen haben, unter den Bedingungen der Gelpolymerisation umgesetzt werden.

**9.** Verwendung der in den Ansprüchen 1 bis 7 beanspruchten Pfropfpolymerisate als Absorptionsmittel für Wasser und wäßrige Lösungen.

**10.** Verwendung der in den Ansprüchen 1 bis 7 beanspruchten Pfropfpolymerisate gemäß Anspruch 9, dadurch gekennzeichnet, daß die Pfropfpolymerisate in Hygieneartikeln wie Windeln, Tampons und Damenbinden eingesetzt werden.

**Patentansprüche für folgenden Vertragsstaat : ES**

**1.** Verfahren zur Herstellung hydrophiler, quellfähiger Pfropfpolymerisate, die in statistischer Verteilung zu 0,5 bis 20 Gew.% aus Resten der allgemeinen Formel I

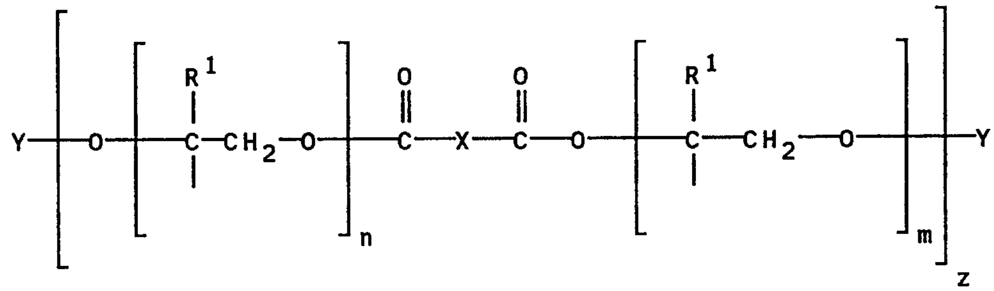 (I)

zu 79 bis 99 Gew.% aus eine saure Gruppe enthaltenden Resten der allgemeinen Formel II

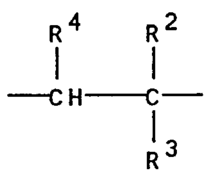 (II)

und zu 0,1 bis 2 Gew.% aus Resten eines Vernetzers, die aus Monomeren mit mindestens zwei olefinisch ungesättigten Doppelbindungen hervorgegangen sind, bestehen, wobei X ($C_1$-$C_6$)-Alkylen, ($C_2$-$C_6$)-Alkenyl, Phenylen oder durch Sulfonyl substituiertes Phenylen, wobei cis-Ethenyl ausgeschlossen ist,
Y Wasserstoff oder

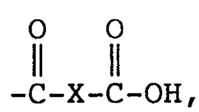

z 1 bis 100,
m und n unabhängig voneinander 2 bis 300,
$R^1$ Wasserstoff oder Methyl,
$R^2$ Wasserstoff, Methyl oder Ethyl,
$R^3$ die Carboxylgruppe, die Sulfonylgruppe, die Phosphonylgruppe, die gegebenenfalls mit Alkanol mit 1 bis 4 Kohlenstoffatomen verestert sein kann, oder eine Gruppe der Formel

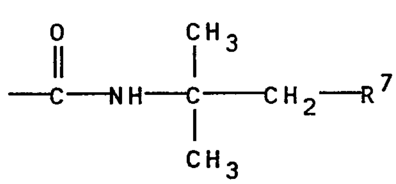

worin $R^7$ für die Sulfonylgruppe oder die Phosphonylgruppe steht, $R^4$ Wasserstoff, Methyl, Ethyl oder

die Carboxylgruppe bedeuten, dadurch gekennzeichnet, daß 0,5 bis 20 Gew.%, vorzugsweise 0,5 bis 15, insbesondere 1 bis 10,5 Gew.%, einer Polyalkylenoxidverbindung der allgemeinen Formel Ia

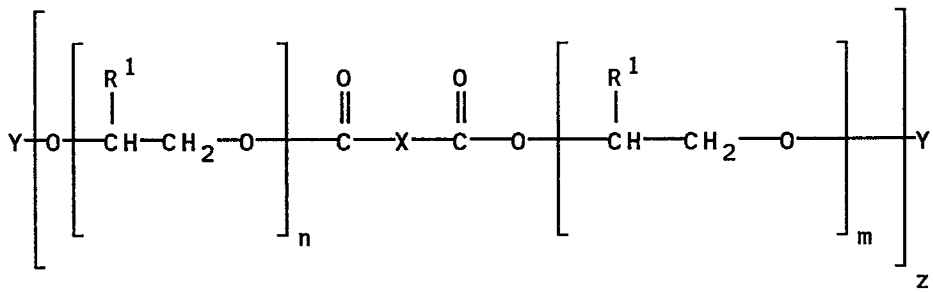

(Ia)

79 bis 99 Gew.%, vorzugsweise 84 bis 99, insbesondere 88 bis 98,5 Gew.%, einer ungesättigten Säure der allgemeinen Formel IIa

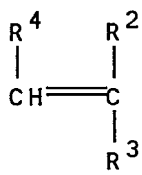

(IIa)

oder ein Alkali-, Ammonium- oder Aminsalz davon und 0,1 bis 2 Gew.%, vorzugsweise 0,1 bis 1,8, insbesondere 0,3 bis 1,5 Gew.%, eines Monomeren mit mindestens zwei olefinisch ungesättigten Doppelbindungen, wobei die Reste $R^1$ bis $R^4$, X, Y und die Zahlen m, n und z die oben genannten Bedeutungen haben, unter den Bedingungen der Gelpolymerisation umgesetzt werden.

**2.** Verfahren gemäß Anspruch 1, dadurch gekennzeichnet, daß die Pfropfpolymerisate zu 1 bis 10,5 Gew.% aus Resten der allgemeinen Formel I, zu 88 bis 98,5 Gew.% aus Resten der allgemeinen Formel II und zu 0,3 bis 1,5 Gew.% aus vernetzenden Strukturen bestehen.

**3.** Verfahren gemäß Ansprüchen 1 und/oder 2, dadurch gekennzeichnet, daß sich die Reste der allgemeinen Formel I hinsichtlich des Restes $R^1$ und/oder der Zahlen m und n voneinander unterscheiden.

**4.** Verfahren nach einem oder mehreren der Ansprüche 1 bis 3, dadurch gekennzeichnet, daß in den Resten der allgemeinen Formel II $R^2$ Wasserstoff oder Methyl, $R^3$ die Carboxylgruppe, die Sulfonylgruppe oder die Phosphonylgruppe und $R^4$ Wasserstoff bedeuten.

**5.** Verfahren nach einem oder mehreren der Ansprüche 1 bis 4, dadurch gekennzeichnet, daß in den Resten der allgemeinen Formel II $R^3$ die Carboxylgruppe bedeutet.

**6.** Verfahren nach einem oder mehreren der Ansprüche 1 bis 5, dadurch gekennzeichnet, daß sich die vernetzenden Strukturen von Monomeren mit mindestens zwei Alkenylgruppen oder mindestens zwei Alkenoylgruppen, insbesondere von Trimethylolpropantriacrylat, Tetraallyloxyethan oder Methylenbisacrylamid ableiten.

**7.** Verfahren nach einem oder mehreren der Ansprüche 1 bis 6, dadurch gekennzeichnet, daß X $(C_1\text{-}C_6)$-Alkylen, 1,4-Phenylen, 1,3-Phenylen oder 5-Sulfo-1,3-phenylen bedeutet.

**8.** Verwendung der nach den Verfahren der Ansprüche 1 bis 7 hergestellten Pfropfpolymerisate als Absorptionsmittel für Wasser und wäßrige Lösungen.

**9.** Verwendung der nach den Verfahren der Ansprüche 1 bis 7 hergestellten Pfropfpolymerisate gemäß Anspruch 8, dadurch gekennzeichnet, daß die Pfropfpolymerisate in Hygieneartikeln wie Windeln,

Tampons und Damenbinden eingesetzt werden.

**Claims**

**Claims for the following Contracting States : BE, CH, DE, DK, FR, GB, IT, LI, NL, SE**

1. Hydrophilic, swellable graft copolymers composed, in random distribution, of 0.5 to 20% by weight of radicals of the general formula I

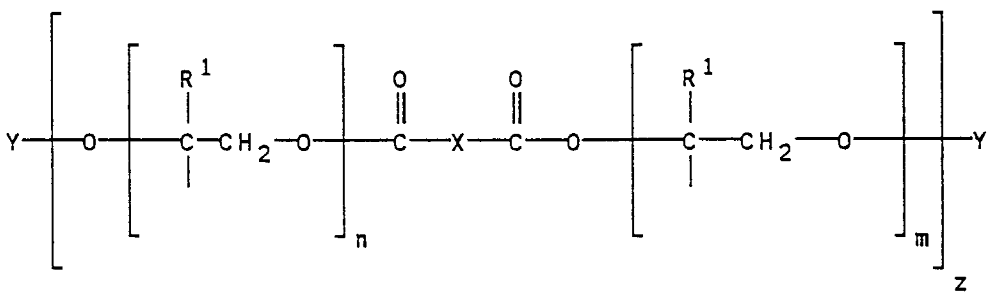

(I)

of 79 to 99% by weight of radicals containing an acidic group, of the general formula II

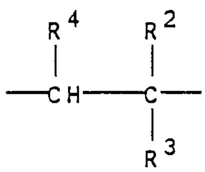

(II)

and of 0.1 to 2% by weight of radicals of a crosslinking agent which are derived from monomers having at least two olefinically unsaturated double bonds, where
X denotes ($C_1$-$C_6$)-alkylene, ($C_2$-$C_6$)-alkenylene, phenylene or sulphonyl-substituted phenylene, cis-ethylene being excluded,
Y denotes hydrogen or

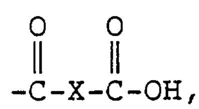

z denotes 1 to 100,
m and n independently of one another denote 2 to 300,
$R^1$ denotes hydrogen or methyl,
$R^2$ denotes hydrogen, methyl or ethyl,
$R^3$ denotes the carboxyl group, the sulphonyl group, the phosphonyl group, which may optionally be esterified with an alkanol having 1 to 4 carbon atoms, or denotes a group of the formula

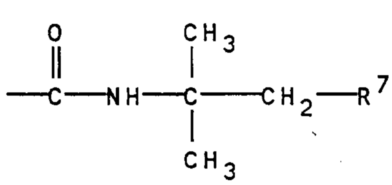

in which $R^7$ represents the sulphonyl group or the phosphonyl group, $R^4$ denotes hydrogen, methyl,

ethyl or the carboxyl group.

2. Graft copolymers according to Claim 1, characterized in that they are composed of 1 to 10.5 % by weight of radicals of the general formula I, 88 to 98.5 % by weight of radicals of the general formula II and 0.3 to 1.5 % by weight of crosslinking structures.

3. Graft copolymers according to Claims 1 and/or 2, characterized in that the radicals of the general formula I differ with regard to the radical $R^1$ and/or the numbers m and n.

4. Graft copolymers according to one or more of Claims 1 to 3, characterized in that the radical $R^2$ in the formula II denotes hydrogen or methyl, the radical $R^3$ denotes the carboxyl group, the sulphonyl group or the phosphonyl group and the radical $R^4$ denotes hydrogen.

5. Graft copolymers according to one or more of Claims 1 to 4, characterized in that the radical $R^3$ of the general formula II denotes the carboxyl group.

6. Graft copolymers according to one or more of Claims 1 to 5, characterized in that the crosslinking structures are derived from monomers having at least two alkenyl groups or at least two alkenoyl groups, in particular from trimethylolpropane triacrylate, tetraallyloxyethane or methylenebisacrylamide.

7. Graft copolymers according to one or more of Claims 1 to 6, characterized in that X denotes $(C_1-C_6)$-alkylene, 1,4-phenylene, 1,3-phenylene or 5-sulpho-1,3-phenylene.

8. Process for the preparation of the graft copolymers claimed in Claims 1 to 7, characterized in that 0.5 to 20 % by weight, preferably 0.5 to 15, in particular 1 to 10.5 % by weight of a polyalkylene oxide compound of the formula Ia

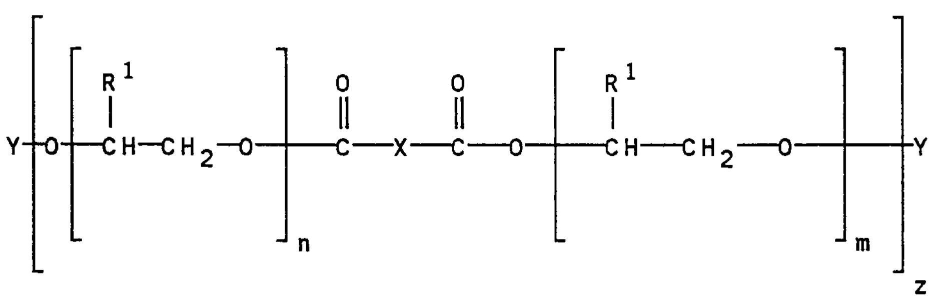

(Ia)

79 to 99 % by weight, preferably 84 to 99, in particular 88 to 98.5 % by weight of an unsaturated acid of the general formula IIa

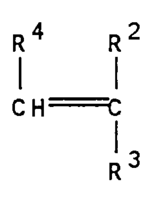

(IIa)

or an alkali metal salt, ammonium salt or amine salt thereof and 0.1 to 2 % by weight, preferably 0.1 to 1.8, in particular 0.3 to 1.5 % by weight of a monomer having at least two olefinically unsaturated double bonds, where the radicals $R^1$ to $R^4$, X, Y and the numbers m, n and z have the meanings given above, are reacted under the conditions of gel polymerization.

9. The use of the graft copolymers claimed in Claims 1 to 7 as absorbents for water and aqueous solutions.

10. The use according to Claim 9 of the graft copolymers claimed in Claims 1 to 7, characterized in that the graft copolymers are used in sanitary items such as diapers, tampons and sanitary towels.

**Claims for the following Contracting State : ES**

1. Process for preparing hydrophilic, swellable graft copolymers composed, in random distribution, of 0.5 to 20% by weight of radicals of the general formula I

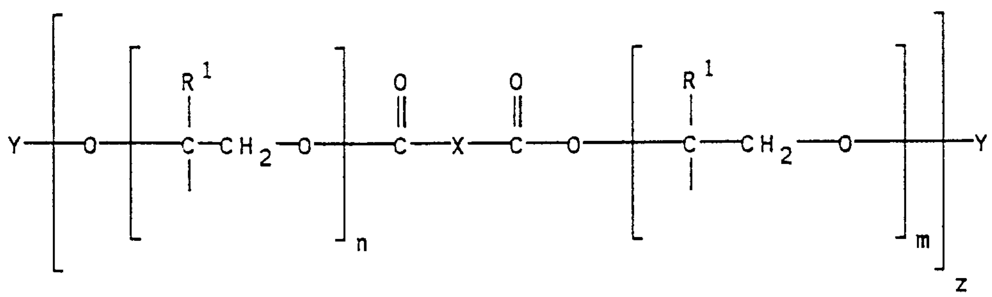 (I)

of 79 to 99% by weight of radicals containing an acidic group, of the general formula II

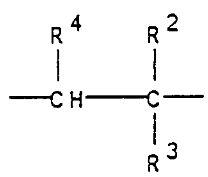 (II)

and of 0.1 to 2% by weight of radicals of a crosslinking agent which are derived from monomers having at least two olefinically unsaturated double bonds, where
X denotes ($C_1$-$C_6$)-alkylene, ($C_2$-$C_6$)-alkenylene, phenylene or sulphonyl-substituted phenylene, cis-ethylene being excluded,
Y denotes hydrogen or

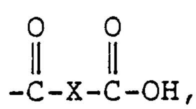

z denotes 1 to 100,
m and n independently of one another denote 2 to 300,
$R^1$ denotes hydrogen or methyl,
$R^2$ denotes hydrogen, methyl or ethyl,
$R^3$ denotes the carboxyl group, the sulphonyl group, the phosphonyl group, which may optionally be esterified with an alkanol having 1 to 4 carbon atoms, or denotes a group of the formula

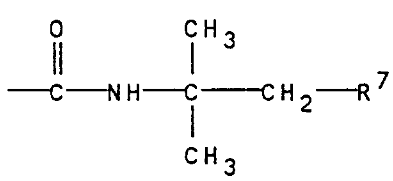

in which $R^7$ represents the sulphonyl group or the phosphonyl group, $R^4$ denotes hydrogen, methyl, ethyl or the carboxyl group, characterized in that 0.5 to 20 % by weight, preferably 0.5 to 15, in particular 1 to 10.5 % by weight of a polyalkylene oxide compound of the formula Ia

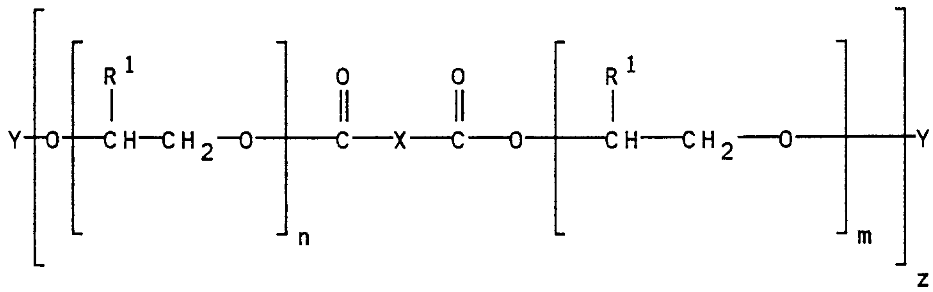

(Ia)

79 to 99 % by weight, preferably 84 to 99, in particular 88 to 98.5 % by weight of an unsaturated acid of the general formula IIa

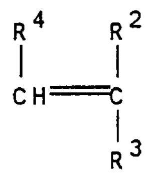

(IIa)

or an alkali metal salt, ammonium salt or amine salt thereof and 0.1 to 2 % by weight, preferably 0.1 to 1.8, in particular 0.3 to 1.5 % by weight of a monomer having at least two olefinically unsaturated double bonds, where the radicals $R^1$ to $R^4$, X, Y and the numbers m, n and z have the meanings given above, are reacted under the conditions of gel polymerization.

**2.** Process according to Claim 1, characterized in that the graft polymers are composed of 1 to 10.5 % by weight of radicals of the general formula I, 88 to 98.5 % by weight of radicals of the general formula II and 0.3 to 1.5 % by weight of crosslinking structures.

**3.** Process according to Claims 1 and/or 2, characterized in that the radicals of the general formula I differ with regard to the radical $R^1$ and/or the numbers m and n.

**4.** Process according to one or more of Claims 1 to 3, characterized in that the radical $R^2$ in the formula II denotes hydrogen or methyl, the radical $R^3$ denotes the carboxyl group, the sulphonyl group or the phosphonyl group and the radical $R^4$ denotes hydrogen.

**5.** Process according to one or more of Claims 1 to 4, characterized in that the radical $R^3$ of the general formula II denotes the carboxyl group.

**6.** Process according to one or more of Claims 1 to 5, characterized in that the crosslinking structures are derived from monomers having at least two alkenyl groups or at least two alkenoyl groups, in particular from trimethylolpropane triacrylate, tetraallyloxyethane or methylenebisacrylamide.

**7.** Process according to one or more of Claims 1 to 6, characterized in that X denotes $(C_1\text{-}C_6)$-alkylene, 1,4-phenylene, 1,3-phenylene or 5-sulpho-1,3-phenylene.

**8.** The use of the graft copolymers prepared according to a process of Claims 1 to 7 as absorbents for water and aqueous solutions.

**9.** The use according to Claim 8 of the graft copolymers claimed in Claims 1 to 7, characterized in that the graft copolymers are used in sanitary items such as diapers, tampons and sanitary towels.

**Revendications**

**Revendications pour les Etats contractants suivants : BE, CH, DE, DK, FR, GB, IT, LI, NL, SE**

1. Polymères greffés gonflables, hydrophiles, qui consistent en répartition statistique en 0,5 à 20% en poids de radicaux de formule générale I

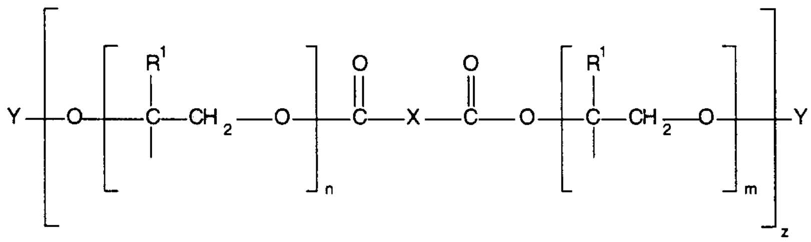

(I)

en 79 à 99% en poids en un radical contenant un groupe acide, de formule générale II

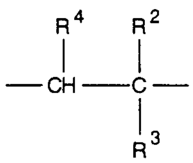

(II)

et en 0,1 à 2% en poids de radicaux provenant d'un agent réticulant, qui proviennent de monomères comportant au moins deux doubles liaisons à insaturation oléfinique, et dans lesquels X représente un radical ($C_1$-$C_6$)-alkylène, ($C_2$-$C_6$)-alkénylène, phénylène ou phénylène substitué par des groupes sulfonyle, le radical cis-éthènylène étant exclu,
Y représente l'atome d'hydrogène ou le groupe

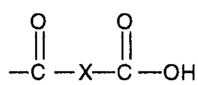

z représente une valeur comprise entre 1 et 100,
m et n représentent indépendamment l'un de l'autre une valeur comprise entre 2 et 300,
$R_1$ représente un atome d'hydrogène ou un groupe méthyle,
$R_2$ représente un atome d'hydrogène, un groupe méthyle ou un groupe éthyle,
$R_3$ représente un groupe carboxy, sulfonyle, phosphonyle, qui peut être éventuellement estérifié par un alcanol comportant de 1 à 4 atomes de carbone, ou un groupe de formule

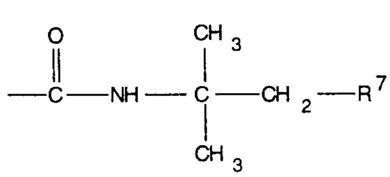

dans laquelle $R_7$ représente le groupe sulfonyle ou le groupe phosphonyle $R_4$ représente l'atome d'hydrogène, un groupe méthyle, éthyle ou le groupe carboxy.

2. Polymères greffés selon la revendication 1, caractérisé en ce qu'ils consistent en 1 à 10,5% en poids de radicaux de formule générale I, 88 à 98,5% en poids de radicaux de formule générale II et 0,3 à 1,5% en poids de structures réticulées.

**3.** Polymères greffés selon la revendication 1 et/ou 2, caractérisés en ce que les radicaux de formule générale I se distinguent les uns des autres au regard des radicaux $R_1$ et/ou des valeurs m et n.

**4.** Polymères greffés selon une ou plusieurs des revendications 1 à 3, caractérisés en ce que dans les radicaux de formule générale II, $R_2$ représente l'hydrogène ou un groupe méthyle, $R_3$ représente un groupe carboxy, un groupe sulfonyle ou un groupe phosphonyle et $R_4$ représente un atome d'hydrogène.

**5.** Polymères greffés selon une ou plusieurs des revendications 1 à 4, caractérisés en ce que dans les radicaux de formule générale II, $R_3$ représente un groupe carboxy.

**6.** Polymères greffés selon une ou plusieurs des revendications 1 à 5, caractérisés en ce que les structures réticulées dérivent de monomères comportant au moins deux groupes alkényle ou au moins deux groupes alkénoyle, en particulier de triacrylate de triméthylolpropane, de tetraallyloxyéthane ou de méthylènebisacrylamide.

**7.** Polymères greffés selon une ou plusieurs des revendications 1 à 6, caractérisés en ce que X représente un groupe $(C_1\text{-}C_6)$-alkylène, 1,4-phénylène, 1,3-phénylène ou 5-sulfo-1,3-phénylène.

**8.** Procédé de préparation des polymères greffés selon les revendications 1 à 7, caractérisé en ce que l'on fait réagir dans les conditions de la polymérisation en gel, de 0,5 à 20% en poids, de préférence de 0,5 à 15, en particulier de 1 à 10,5% en poids, d'un composé de type oxyde de polyalkylène de formule générale Ia

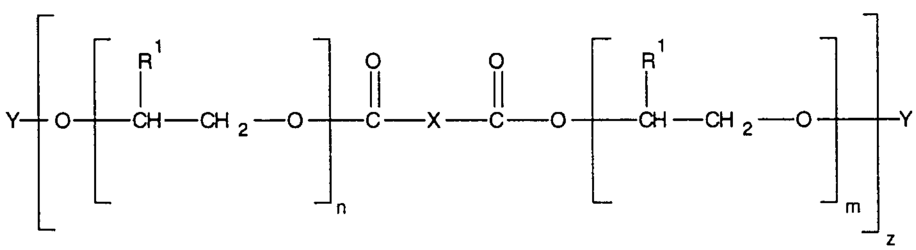

(Ia)

79 à 99% en poids, de préférence 84 à 99, en particulier 88 à 98,5% en poids, d'un acide insaturé de formule générale IIa

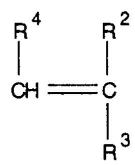

(IIa)

ou un sel de métal alcalin, d'ammonium ou d'amine, de cet acide, et 0,1 à 2% en poids, de préférence de 0,1 à 1,8, en particulier de 0,3 à 1,5% en poids, d'un monomère comportant au moins deux doubles liaisons à insaturation oléfinique, les radicaux $R_1$ à $R_4$, X et Y, et les nombres m, n et z étant tels que définis ci-dessus.

**9.** Utilisation des polymères greffés selon les revendications 1 à 7, comme agents d'absorption de l'eau et des solutions aqueuses.

**10.** Utilisation des polymères greffés selon les revendications 1 à 7, selon la revendication 9, caractérisée en ce que l'on incorpore les polymères greffés dans des articles d'hygiène comme des couches pour bébés, des tampons et des articles d'hygiène féminine.

**Revendications pour l'Etat contractant suivant : ES**

1. Procédé de préparation de polymères greffés gonflables, hydrophiles, qui consistent en répartition statistique en 0,5 à 20% en poids de radicaux de formule générale I

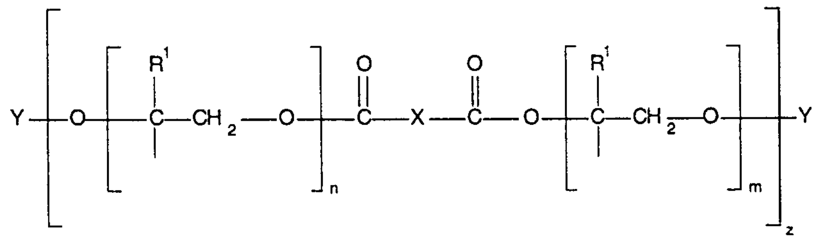

(I)

en 79 à 99% en poids en un radical contenant un groupe acide, de formule générale II

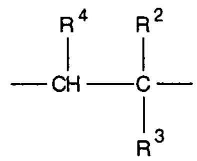

(II)

et en 0,1 à 2% en poids de radicaux provenant d'un agent réticulant, qui proviennent de monomères comportant au moins deux doubles liaisons à insaturation oléfinique, et dans lesquels X représente un radical ($C_1$-$C_6$)-alkylène, ($C_2$-$C_6$)-alkénylène, phénylène ou phénylène substitué par des groupes sulfonyle, le radical cis-éthènylène étant exclu,
Y représente l'atome d'hydrogène ou le groupe

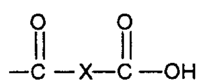

z représente une valeur comprise entre 1 et 100,
m et n représentent indépendamment l'un de l'autre une valeur comprise entre 2 et 300,
$R_1$ représente un atome d'hydrogène ou un groupe méthyle,
$R_2$ représente un atome d'hydrogène, un groupe méthyle ou un groupe éthyle,
$R_3$ représente un groupe carboxy, sulfonyle, phosphonyle, qui peut être éventuellement estérifié par un alcanol comportant de 1 à 4 atomes de carbone, ou un groupe de formule

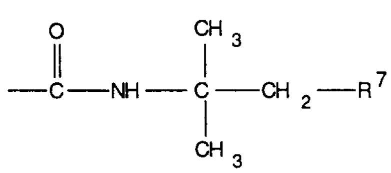

dans laquelle $R_7$ représente le groupe sulfonyle ou le groupe phosphonyle $R_4$ représente l'atome d'hydrogène, un groupe méthyle, éthyle ou le groupe carboxy, caractérisé en ce que l'on fait réagir dans les conditions de la polymérisation en gel de 0,5 à 20% en poids, de préférence de 0,5 à 15, en particulier de 1 à 10,5% en poids, d'un composé de type oxyde de polyalkylène de formule générale Ia

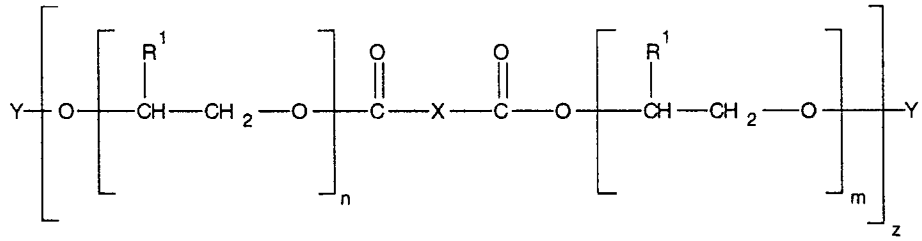

(Ia)

79 à 99% en poids, de préférence 84 à 99, en particulier 88 à 98,5% en poids, d'un acide insaturé de formule générale IIa

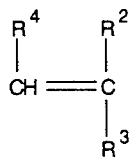

(IIa)

ou un sel de métal alcalin, d'ammonium ou d'amine, de cet acide, et 0,1 à 2% en poids, de préférence de 0,1 à 1,8, en particulier de 0,3 à 1,5% en poids, d'un monomère comportant au moins deux doubles liaisons à insaturation oléfinique, les radicaux $R_1$ à $R_4$, X et Y, et les nombres m, n et z étant tels que définis ci-dessus.

**2.** Procédé selon la revendication 1, caractérisé en ce que les polymères greffés consistent en 1 à 10,5% en poids de radicaux de formule générale I, 88 à 98,5% en poids de radicaux de formule générale II et 0,3 à 1,5% en poids de structures réticulées.

**3.** Procédé selon la revendication 1 et/ou 2, caractérisé en ce que les radicaux de formule générale I se distinguent les uns des autres au regard des radicaux $R_1$ et/ou des valeurs m et n.

**4.** Procédé selon une ou plusieurs des revendications 1 à 3, caractérisé en ce que dans les radicaux de formule générale II, $R_2$ représente l'hydrogène ou un groupe méthyle, $R_3$ représente un groupe carboxy, un groupe sulfonyle ou un groupe phosphonyle et $R_4$ représente un atome d'hydrogène.

**5.** Procédé selon une ou plusieurs des revendications 1 à 4, caractérisé en ce que dans les radicaux de formule générale II, $R_3$ représente un groupe carboxy.

**6.** Procédé selon une ou plusieurs des revendications 1 à 5, caractérisé en ce que les structures réticulées dérivent de monomères comportant au moins deux groupes alkényle ou au moins deux groupes alkénoyle, en particulier de triacrylate de triméthylolpropane, de tetraallyloxyéthane ou de méthylènebisacrylamide.

**7.** Procédé selon une ou plusieurs des revendications 1 à 6, caractérisé en ce que X représente un groupe ($C_1$-$C_6$)-alkylène, 1,4-phénylène, 1,3-phénylène ou 5-sulfo-1,3-phénylène.

**8.** Utilisation des polymères greffés préparés selon le procédé des revendications 1 à 7 comme agent d'absorption de l'eau et de solutions aqueuses.

**9.** Utilisation des polymères greffés préparés selon le procédé des revendications 1 à 7, selon la revendication 8, caractérisée en ce que les polymères greffés sont incorporés dans des articles d'hygiène comme des couches pour bébés, des tampons et des articles d'hygiène féminine.